# FASCICULE DE BREVET EUROPEEN

(11) **EP 1 804 700 B1**
(45) Date de publication et mention de la délivrance du brevet: **20.08.2008**
(21) Numéro de dépôt: 05809108.3
(22) Date de dépôt: 06.10.2005
(51) Int. Cl.: A61B 17/86, A61B 17/80

(54) **VIS POUR DISPOSITIF DE COAPTATION DE FRAGMENTS OU PARTIES D'OS, UTILISANT UN EFFET DE COMPRESSION.**
SCHRAUBE FÜR EINE VORRICHTUNG FÜR DIE KOAPTATION VON KNOCHENFRAGMENTEN ODER TEILEN MIT KOMPRESSIONSWIRKUNG
SCREW FOR DEVICE FOR COAPTATION OF BONE FRAGMENTS OR PARTS USING COMPRESSION EFFECT

(30) Priorité: 07.10.2004 FR 0410583
(43) Date de publication de la demande: 11.07.2007
(73) Titulaire: Biotech International (SARL), 13300 Salon de Provence (FR)
(72) Inventeur: COLOMBIER, Jean-Alain Nouvelle Clinique de l'Union, F-31240 Saint Jean (FR); TCHURUKDICHIAN, Alain, F-21000 Dijon (FR); HOUVET, Patrick, F-92100 Boulogne (FR)
(74) Mandataire: Marek, Pierre
(86) Numéro de dépôt international: PCT/FR2005/002469
(87) Numéro de publication internationale: WO 2006/037898

(56) Documents cités:
- WO-A-20/04032751
- FR-A- 2 760 628
- US-A- 4 463 753
- US-A1- 2002 016 594
- US-B1- 6 306 140

## Description

Vis pour dispositif de coaptation de fragments ou parties d'os, utilisant un effet de compression.

La présente invention concerne une vis pour dispositif de coaptation de fragments ou parties d'os, utilisant un effet de compression connu sous le nom d"'effet DCP" (Dynamic Compression Plate). Elle vise également le dispositif d'ostéosynthèse autobloquant utilisant de telles vis.

La coaptation de fragments ou parties d'os avec effet DCP, destinée à réparer une fracture ou à réaliser une ostéotomie est généralement obtenue au moyen de plaques d'ostéosynthèse pourvues de trous pour le passage des vis de fixation desdites plaques sur les fragments ou parties d'os à rapprocher et à compresser pour favoriser la synthèse de ces derniers. Les vis utilisées pour la fixation de la plaque comportent une partie distale filetée et une partie proximale ou tête sphérique. En vissant cette vis avec un léger décalage vers l'extérieur par rapport à l'axe des trous de la plaque, la tête sphérique vient s'appuyer contre une portion du bord du trou et provoque un mouvement relatif entre la plaque et la vis (et l'os solidaire de celle-ci). En procédant de la sorte de part et d'autre de la fracture ou séparation à réduire, on assure un rapprochement et une mise en compression des deux fragments ou parties d'os reliés par la plaque.

Toutefois, ces vis à tête sphérique ne sont pas auto-bloquantes en position, de sorte qu'il est nécessaire de mettre en place un organe anti-recul pour empêcher leur dévissage ; cette obligation a naturellement des répercussions défavorables sur la durée et sur le coût des interventions de chirurgie réparatrice.

On connaît des vis d'ostéosynthèse autobloquantes comportant une partie distale pourvue d'un filetage cylindrique, une partie proximale ou tête dotée d'un filetage conique, et un tronçon cylindrique lisse intermédiaire disposé entre leurs parties distale et proximale et se raccordant directement à leur partie proximale. Une vis de ce genre dotée de particularités propres, est, par exemple, décrite dans le document FR-2.760.628, et dans le document US-6.306.140.

On connaît aussi un dispositif de coaptation de fragments ou parties d'os comprenant une plaque d'ostéosynthèse pourvue de trous pour le passage de la tige d'organes de fixation et des vis taraudeuses comportant les caractéristiques susmentionnées.

De manière avantageuse, cette plaque est exécutée, au moins dans les zones délimitant les trous de passage des vis, dans un matériau présentant des propriétés mécaniques autorisant un auto-taraudage du pourtour desdits trous au moyen de vis taraudeuses ; le pourtour des trous étant, par exemple, constitué par un insert exécuté en matière plastique biocompatible. Une telle plaque d'ostéosynthèse est décrite dans le document WO-2004/032751.

Il est possible d'effectuer une ostéosynthèse avec effet de compression ou effet DCP au moyen de vis décrites dans le document FR-2.760.628 ou dans le document US-6.306.140, et d'une plaque décrite dans le document WO-2004/032751, mais l'obtention de cet effet n'est cependant pas assurée sans problème.

En effet, le raccordement direct du tronçon cylindrique intermédiaire lisse avec la tête conique filetée des vis crée une sorte de marche d'escalier saillante et coupante qui, lors du vissage, risque d'endommager le bord des trous de la plaque en nuisant ainsi à l'effet d'autoblocage desdites vis lorsque leur tête se trouve serrée dans la matière constituant le pourtour desdits trous. D'autre part, cette "marche d'escalier" saillante constitue un obstacle contrariant le mouvement longitudinal de glissement des vis lors de leur vissage, ladite marche venant buter contre le bord supérieur du trou.

Un objet de la présente invention est de remédier à cet inconvénient, sans générer de complications constructives ou d'utilisation, de sorte à mettre à la disposition des praticiens, une vis et un dispositif d'ostéosynthèse autobloquant permettant la coaptation des fragments ou parties d'os avec effet DCP.

Selon l'invention, cet objectif est réalisé au moyen d'une vis d'ostéosynthèse comportant une partie distale pourvue d'un filetage cylindrique, une partie proximale ou tête dotée d'un filetage taraudeur conique et un tronçon lisse intermédiaire disposé entre ses parties distale et proximale, cette vis étant remarquable en ce que ledit tronçon lisse intermédiaire est constitué par une succession de surfaces lisses de révolution tangentes les unes aux autres, lesdites surfaces lisses de révolution comprenant, successivement, à partir de la partie distale filetée une surface cylindrique, une surface torique et une surface sphérique par l'intermédiaire de laquelle ledit tronçon lisse se raccorde à la partie proximale de la vis.

Le dispositif de coaptation de fragments ou parties d'os selon l'invention comprend des vis taraudeuses comportant les caractéristiques susmentionnées et une plaque d'ostéosynthèse pourvue de trous pour le passage de ces vis, cette plaque d'ostéosynthèse étant, de préférence, du type décrit dans le document WO-2004/032751.

Grâce à la conformation du tronçon lisse intermédiaire des vis selon l'invention, il est possible d'obtenir une fixation autobloquante des plaques d'ostéosynthèse, avec un effet de compression ou effet DCP et cela en évitant d'endommager le bord des trous d'une plaque d'ostéosynthèse, lors du vissage des vis d'ostéosynthèse.

Les buts, caractéristiques et avantages ci-dessus, et d'autres encore, ressortiront mieux de la description qui suit et des dessins annexés dans lesquels :

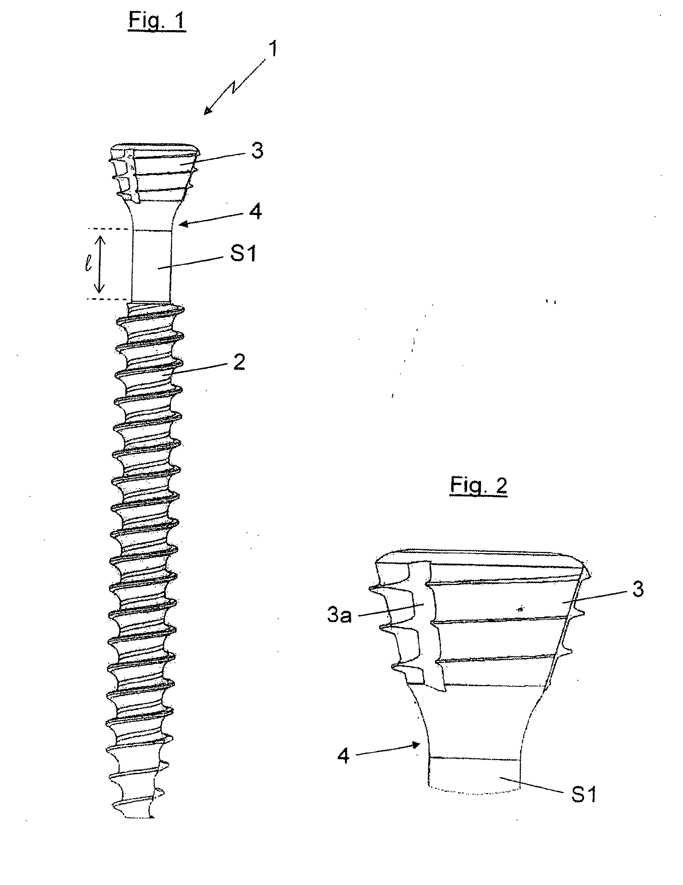

La figure 1 est une vue en élévation d'une vis d'ostéosynthèse selon l'invention.
La figure 2 est une vue en élévation et à plus grande échelle de la partie proximale de cette vis.
La figure 3 est une vue à caractère schématique et à échelle très agrandie, illustrant la configuration de la partie proximale du tronçon lisse intermédiaire des vis selon l'invention.

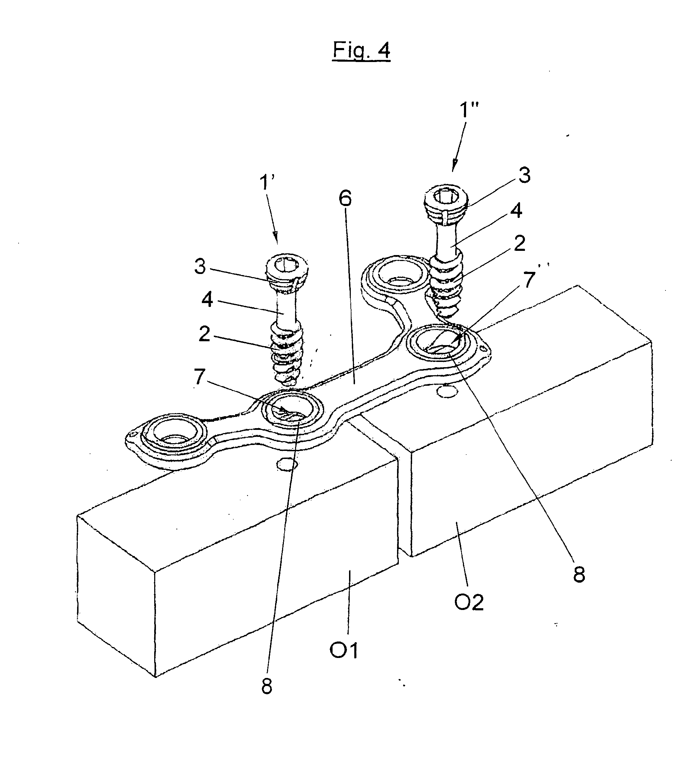

La figure 4 est une vue en perspective éclatée et à caractère schématique d'un exemple de dispositif d'ostéosynthèse autobloquant utilisable pour réaliser la coaptation de fragments d'os avec un effet DCP.
Les figures 5A à 5E illustrent, par des vues en coupe et à caractère schématique, le dispositif d'ostéosynthèse autobloquant selon l'invention et les différentes étapes de sa fixation sur des fragments ou parties d'os, avec effet DCP.

On se reporte auxdits dessins pour décrire un exemple intéressant, quoique nullement limitatif de réalisation de la vis et du dispositif d'ostéosynthèse autobloquant selon l'invention.

Cette vis 1 comporte une partie distale 2 pourvue d'un filetage cylindrique, une partie proximale ou tête dotée d'un filetage taraudeur conique 3 dont le diamètre croît en direction de l'extrémité proximale de ladite vis, et un tronçon lisse intermédiaire 4 disposé entre les parties distale 2 et proximale 3 filetées de cette dernière. Le filetage conique de la partie proximale 3 peut comporter une ou plusieurs entailles 3a orientées suivant l'une ou plusieurs de ses génératrices, et conférant à la tête conique 3 une action d'auto-taraudage lors du vissage.

Une vis de ce genre qui peut être exécutée en titane ou en inox ou autre matériau biocompatible, est, par exemple, décrite dans le document FR-2.760.628.

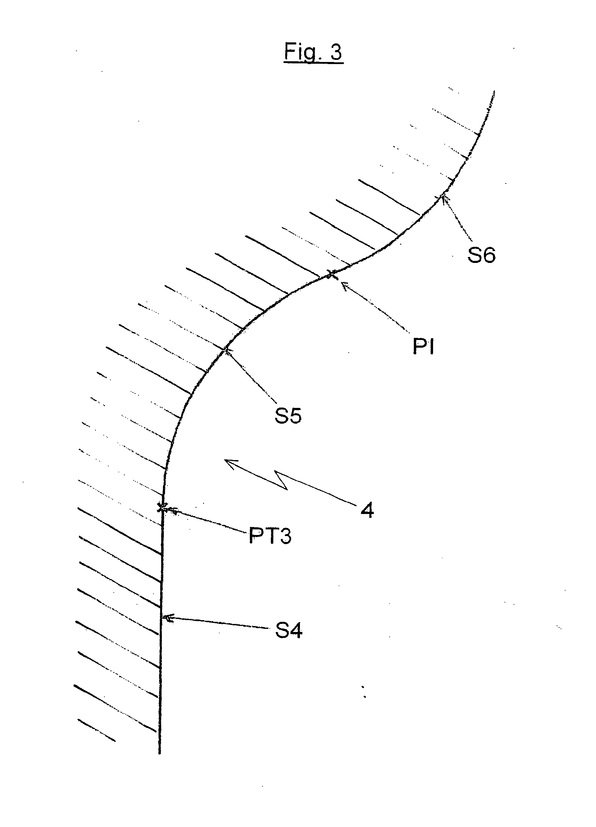

Selon une disposition caractéristique de l'invention, le tronçon lisse intermédiaire 4 de la vis présente une succession de surfaces lisses de révolution S4, S5, S6 (figure 3).

Comme le montre plus précisément la figure 3, le tronçon lisse intermédiaire 4 de la vis présente successivement, à partir de la partie distale filetée 2 de celle-ci : une surface cylindrique S4, une surface torique S5 et une surface sphérique S6 par l'intermédiaire de laquelle ledit tronçon 4 se raccorde à la partie proximale 3 de ladite vis. La référence PT3 indique le point de tangence et la référence PI le point d'inflexion de la génératrice continue de ces surfaces.

Outre des vis présentant la disposition caractéristique susmentionnée, le dispositif d'ostéosynthèse selon l'invention comprend une plaque 6 munie de trous traversants 7, cette plaque pouvant présenter toute taille et conformation adaptées aux cas de réductions de fractures ou de chirurgie restauratrice à traiter.

Cette plaque est avantageusement du type décrit dans le document WO-2004/032.751, c'est-à-dire une plaque exécutée, au moins dans les zones délimitant les trous 7, dans un matériau 8 présentant des propriétés mécaniques permettant un auto-taraudage du pourtour desdits trous au moyen de la tête taraudeuse des vis 1 utilisables pour la fixation de ladite plaque sur la matière osseuse.

De préférence, la plaque 6 est constituée par une plaque composite dont les pourtours 8 des trous 7 sont constitués par des inserts exécutés dans une matière plastique biocompatible, la partie ou surface restante de ladite plaque étant réalisée en métal (par exemple en titane ou en acier inoxydable). Les inserts 8 peuvent être exécutés en un polymère thermoplastique à hautes performances, de préférence en Polyétheréthercétone (PEEK).

La partie cylindrique S4 du tronçon lisse intermédiaire 4 des vis a une longueur I correspondant au moins à l'épaisseur de la plaque 6, à l'emplacement des trous 7.

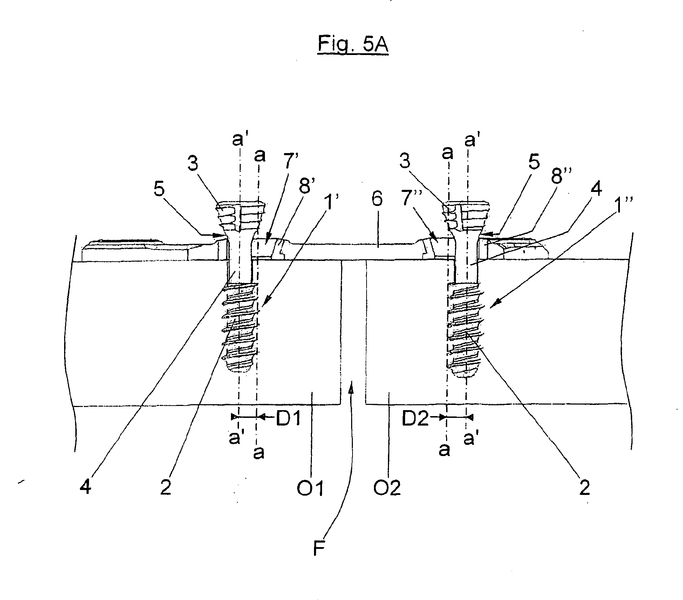

Les figures 5A à 5E illustrent les différentes étapes d'une intervention d'ostéotomie réalisée au moyen des vis et du dispositif selon l'invention.

Après application de la plaque 6 sur les fragments d'os 01 et 02 à réunir, de part et d'autre de la fracture ou coupure F, les vis 1', 1" sont vissées dans lesdits fragments d'os avec un décalage identique D1 ou D2 de leur axe a'-a' par rapport à l'axe a-a des trous 7', 7" de la plaque 6 (figure 5A), jusqu'à ce que leurs surfaces de transition 5 (S5-S6) viennent au contact du bord supérieur de l'insert 8', 8" constituant le pourtour desdits trous.

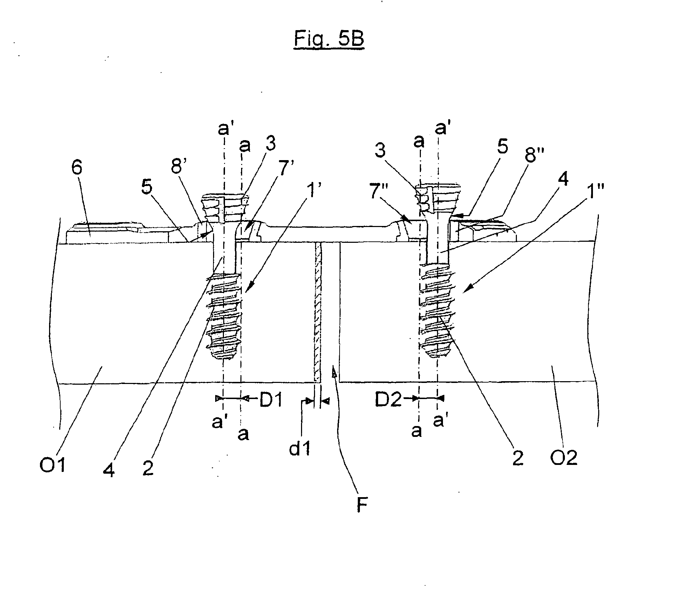

Lors de la poursuite du vissage de l'une des vis (vis 1' selon la figure 5B), la surface de transition 5 (S5-S6) de cette vis glisse sur le bord supérieur 8' du trou 7' qu'elle traverse, en entraînant un premier petit déplacement d1 de la vis 1' et du fragment d'os 01 solidaire de celle-ci, en direction du fragment d'os O2. Dans ce cas, D1 < D2.

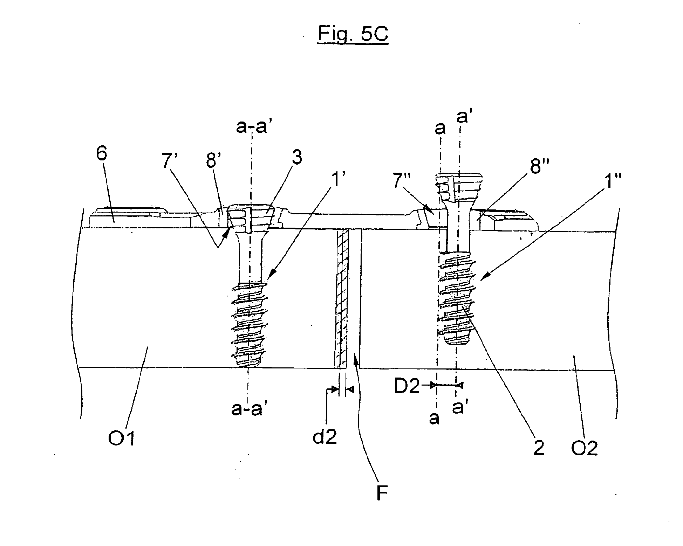

En continuant le vissage de cette vis, sa tête taraudeuse filetée conique 3 s'engage naturellement dans le trou 7' et creuse son propre sillon hélicoïdal récepteur dans le pourtour 8' du trou 7', en entraînant un deuxième petit déplacement d2 de la vis 1' et du fragment d'os 01 en direction du fragment d'os 02 (figure 5C). Dans cette situation, D1 = O.

La somme des déplacements d1 et d2 correspond au décalage initial D1 entre l'axe a'-a' de la vis 1' et l'axe a-a du trou 7'.

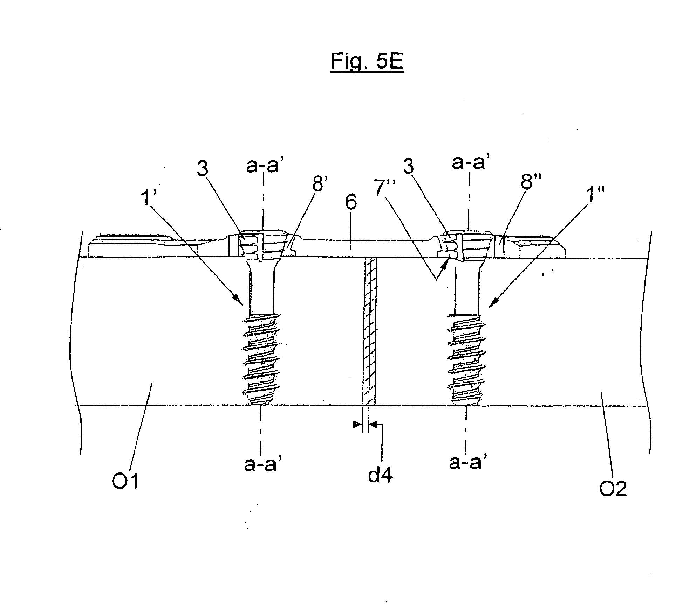

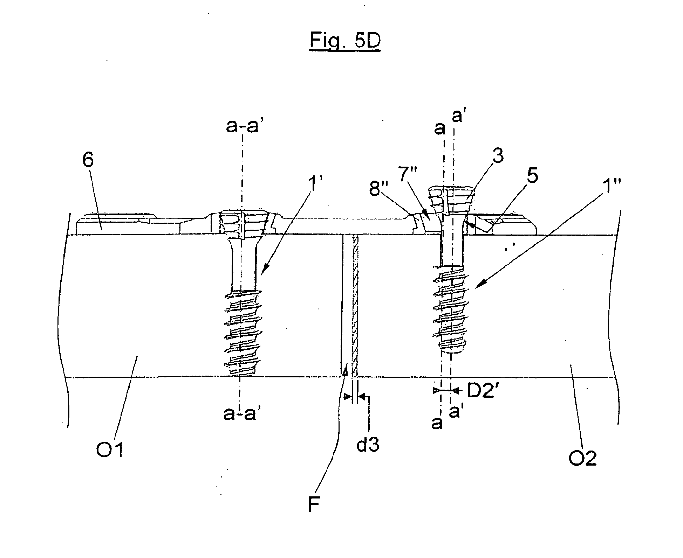

On complète ensuite le vissage de la vis 1" qui, dans un premier temps, entraîne un premier petit déplacement d3 de la vis 1" et du fragment d'os 02 en direction du fragment d'os 01, sous l'action exercée par les surfaces de transition 5 (S5-S6) de ladite vis 1" (figure 5D), de sorte que D2' < D2 ; puis, dans un deuxième temps, le vissage de la tête conique taraudeuse 3 de cette vis dans le pourtour 8" du trou 7", en entraînant un second petit déplacement d4 de cette dernière et du fragment d'os 02 en direction du fragment d'os 01 (figure 5E).

La somme des déplacements d3 et d4 correspond au décalage initial D2 entre l'axe a'-a' de la vis 1" et l'axe a-a du trou 7" de la plaque 6.

En fin d'intervention (figure 5E) les têtes 3 des vis 1' et 1" se trouvent vissées et verrouillées sur la plaque 6 et les deux fragments osseux O1 et O2 sont mis en compression.

## Revendications

1. Vis d'ostéosynthèse pour dispositif de coaptation de fragments ou parties d'os utilisant un effet de compression, comportant une partie distale (2) pourvue d'un filetage cylindrique, une partie proximale ou tête (3) dotée d'un filetage taraudeur conique et un tronçon lisse intermédiaire (4) disposé entre ces parties distale (2) et proximale (3), et constitué par une succession de surfaces lisses de révolution (S4, S5, S6) tangentes les unes aux autres, **caractérisée en ce que** lesdites surfaces comprennent, successivement, à partir de la partie distale filetée (2), une surface cylindrique (S4), une surface torique (S5) et une surface sphérique (S6) par l'intermédiaire de laquelle ledit tronçon (4) se raccorde à la partie proximale (3) de ladite vis.

2. Dispositif de coaptation de fragments ou parties d'os utilisant un effet de compression, **caractérisé en ce qu'**il comprend des vis d'ostéosynthèse (1', 1", ...) selon la revendication 1, et une plaque d'ostéosynthèse (6) munie de trous traversants (7) dont le pourtour (8) est exécuté dans un matériau présentant des propriétés mécaniques permettant un auto-taraudage dudit pourtour (8) par la tête taraudeuse (3) desdites vis.

3. Dispositif selon la revendication 2, **caractérisé en ce que** la partie lisse intermédiaire cylindrique (S1, S4) du tronçon lisse intermédiaire (4) des vis (1) a une longueur (I) correspondant au moins à l'épaisseur de la plaque (6), à l'emplacement des trous (7).

## Claims

1. An osteosynthesis screw for a device for coaptation of bone fragments or parts using a compression effect, comprising a distal portion (2) provided with a cylindrical screwthread, a proximal portion or head (3) provided with a conical tapping screwthread and a smooth intermediate portion (4) disposed between said distal (2) and proximal (3) portions and formed by a succession of smooth revolution surfaces (S4, S5, S6) which are tangential with each other, **characterised in that** said surfaces successively comprise from the screwthreaded distal portion (2) a cylindrical surface (S4), a toric surface (S5) and a spherical surface (S6), by way of which said intermediate portion (4) is connected to the proximal portion (3) of said screw.

2. A device for coaptation of bone fragments or parts using a compression effect **characterised in that** it comprises osteosynthesis screws (1', 1", ...) according to claim 1 and an osteosynthesis plate (6) provided with through holes (7), the periphery (8) of which is made of a material having mechanical properties permitting self-tapping of said periphery (8) by the tapping head (3) of said screws.

3. A device according to claim 2 **characterised in that** the smooth cylindrical intermediate part (S1, S4) of the smooth intermediate portion (4) of the screws (1) is of a length (I) corresponding at least to the thickness of the plate (6) at the location of the holes (7).

## Patentansprüche

1. Osteosyntheseschraube für eine Vorrichtung zur Koaptation von Knochenfragmenten oder -teilen unter Verwendung einer Kompressionswirkung, enthaltend einen distalen Teil (2), der mit einem zylindrischen Gewinde versehen ist, einen proximalen Teil oder Kopf (3), der mit einem ein konisches Innengewinde schneidenden Gewinde ausgestattet ist, und einen dazwischen liegenden glatten Abschnitt (4), der zwischen diesem distalen (2) und diesem proximalen (3) Teil angeordnet ist und von einer Aufeinanderfolge von glatten Rotationsflächen (S4, S5, S6) gebildet wird, die einander berühren, **dadurch gekennzeichnet, dass** die Flächen nacheinander ausgehend von dem distalen Gewindeteil (2) eine zylindrische Fläche (S4), eine wulstförmige Fläche (55) und eine kugelförmige Fläche (56) aufweisen, mittels der sich der Abschnitt (4) an den proximalen Teil (3) der Schraube anschließt.

2. Vorrichtung zur Koaptation von Knochenfragmenten oder -teilen unter Verwendung einer Kompressionswirkung, **dadurch gekennzeichnet, dass** sie Osteosyntheseschrauben (1', 1", ...) nach Anspruch 1 und eine Osteosyntheseplatte (6) umfasst, die mit Durchgangslöchern (7) versehen ist, deren Umfang (8) in einem Material ausgeführt ist, das mechanische Eigenschaften aufweist, die ein selbsttätiges Innengewindeschneiden des Umfangs (8) durch den Innengewindeschneidekopf (3) der Schrauben gestatten.

3. Vorrichtung nach Anspruch 2, **dadurch gekennzeichnet, dass** der glatte dazwischen liegende zylindrische Teil (S1, 54) des dazwischen liegenden glatten Abschnitts (4) der Schrauben (1) eine Länge (1) aufweist, die wenigstens der Dicke der Platte (6) an der Stelle der Löcher (7) entspricht.
